(19)

Europäisches Patentamt

European Patent Office

Office européen des brevets

(11)  **EP 4 338 763 A1**

(12)  # EUROPEAN PATENT APPLICATION

(43) Date of publication:
**20.03.2024   Bulletin 2024/12**

(21) Application number: **22195467.0**

(22) Date of filing: **13.09.2022**

(51) International Patent Classification (IPC):
*A61L 27/22* (2006.01)    *A61L 27/26* (2006.01)
*A61L 27/52* (2006.01)    *A61L 27/56* (2006.01)
*B33Y 10/00* (2015.01)

(52) Cooperative Patent Classification (CPC):
(C-Sets available)
**A61L 27/227; A61L 27/26; A61L 27/52;
A61L 27/56; B33Y 10/00**                      (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fraunhofer-Gesellschaft zur Förderung
der angewandten Forschung e.V.
80686 München (DE)**

(72) Inventors:
 • **SCHMELZER, Christian
   06120 Halle (Saale) (DE)**
 • **FRIEDMANN, Andrea
   06120 Halle (Saale) (DE)**
 • **HEDTKE, Tobias
   06120 Halle (Saale) (DE)**

(74) Representative: **Maiwald GmbH
Elisenhof
Elisenstraße 3
80335 München (DE)**

(54)  ## A PROCESS FOR 3D BIOPRINTING UTILIZING A HYDROGEL COMPRISING A WATER-SOLUBLE ELASTIN DERIVATIVE

(57)   A process for producing a biocompatible three-dimensional article, the process comprising the steps of providing in an elastin derivative provision step a water-soluble elastin derivative, producing in a hydrogel production step a hydrogel for 3D bioprinting comprising said water-soluble elastin derivative, and 3D bioprinting in a bioprinting step the biocompatible three-dimensional article by extrusion of said hydrogel.

EP 4 338 763 A1

(52) Cooperative Patent Classification (CPC): (Cont.)

C-Sets
**A61L 27/26, C08L 89/00;**
**A61L 27/26, C08L 89/06**

## Description

## Technical Field

The invention relates to hydrogels suitable for 3D bioprinting, especially to hydrogels suitable for mimicking the function and mechanical properties of the extracellular matrix of a tissue. The present invention is further concerned with the use of such hydrogels in the process of 3D bioprinting, to bioinks and biomaterial inks made from such hydrogels, and to articles made from such hydrogels by 3D bioprinting.

## Background

Three-dimensional (3D) printing, also known as additive manufacturing, is an innovative way of creating three-dimensional objects via selective deposition of a material such as polymers or ceramics based on computer-designed models and by usage of computer-controlled deposition modules. 3D printing technologies may potentially be used in medical applications and treatment, i.e. by providing customized tissue-based objects to be used in said treatments. Especially in view of worldwide populational growth and aging, it may provide a quick and highly customizable method for treatment by provision of engineered tissue and objects thereof. Such engineered tissue replicates provided via 3D printing could be used in regenerative medicine, a branch of medicine which aims at restoring the functionality of damaged or diseased body parts.

To achieve such three-dimensional biocompatible structures, living cells can be introduced in the material of the printed object, either during or after the printing process. The latter can be achieved by the top-down approach, in which a cell scaffold made up of biocompatible polymers is subsequently seeded with cells. However, in such a method, the control over spatial cell distribution, cell density and tissue vascularization is limited. Consequently, this method is not suitable for printing complex structures such as organs.

Hence, more complex structures can be produced by the second method, which implements the usage of materials already comprising cells in the 3D printing process. This method is usually denominated as 3D bioprinting and the printing materials suitable for 3D bioprinting are usually referred to as bioinks.

The materials used in bioprinting should be biocompatible and processable according to the implemented 3D printing method. In most of the 3D printing methods, processability of the material is achieved by extrudability. Ideally, the used materials should also simulate the function and mechanical properties of the extracellular matrix (ECM) of a chosen tissue after printing. This includes providing cues for cell proliferation, transport of nutrients and signaling agents. Furthermore, such materials ideally degrade to allow healthy tissue to grow.

Printable formulations are typically based on polymer hydrogels, which are suitable due to the intricate architecture mimicking the ECM, high water content, and tailorable properties, depending on their composition. They usually consist of a network of hydrophilic polymers, which may be either of natural (e.g. collagen, gelatin, cellulose, etc.) or synthetic (e.g. poly(ethylene glycol) - PEG, poly(2-hydroxyethylmethacrylate) - PHEMA, etc.) origin.

Beside material properties, the intended function of the produced three-dimensional structures also limit the processing conditions such as temperature, humidity and pressure. This limitation is particularly caused by the presence of living cells to preserve their function. Hence, only a few 3D printing methods can be used, i.e stereolithography-, inkjet- and extrusion-based printing.

Stereolithography-based printing implements selective light exposure of a liquid photo-reactive hydrogel precursor, thereby creating the layers of an object during light-driven polymerization. On the other hand, inkjet-based printing resembles traditional printing on paper, where an actuator driven by stimuli such as heat, pressure and even laser controls the deposition of droplets onto a substrate. Both techniques are challenging for 3D bioprinting applications due to the lack of suitable materials with biocompatibility. Additionally, stereolithography is inflexible for printing multiple formulations in a single object, while inkjet-printing poses challenges for controlling droplet formation and stability once they are deposited.

Hence, typically extrusion-based 3D printing is applied in 3D bioprinting. Extrusion-based 3D printing utilizes ejection of a material through a nozzle, which is usually mobile in three dimensions, onto a platform. To avoid issues such as nozzle clogging, ink drying and high shear rates, which can cause cell agglomeration and damage in cell-laden materials, rheological characterization of the used material is usually required. To improve printing quality and stability of the printed objects, covalent crosslinking, such as photocrosslinking, is typically used. In covalently crosslinked structures, the polymer chains are bound together by multiple covalent bonds, thereby increasing mechanical strength. Moreover, also physical crosslinks are known, which rely solely on intermolecular interactions. Both types of crosslinks can be combined to introduce a dynamical responsivity to external stimuli in the printed objects.

Hence, the choice of used material, especially in view of combining suitable mechanical properties and bioactivity, is one of the biggest challenges in 3D bioprinting. Recently, elastin has emerged as a candidate for 3D bioprinting. Elastin is present as a structural protein in the ECM, thereby providing elasticity to the tissues and taking part in several physiological processes via its degradation subproducts. Elastin is an insoluble protein that is *in vivo* formed by the polymerization of tropoelastin, a soluble polypeptide precursor, through crosslinking of its lysine residues. Elastin allows for highly tunable hydrogel formulations in view of the resulting physical properties of the printed article. Especially the elastic modulus can be tuned and enhanced by elastin in a way it cannot be achieve using other typical natural polymers used in hydrogels for 3D bioprinting, such as gelatin or collagen.

**Summary of the invention**

Hence, for 3D bioprinting, a way of achieving properties of elastin in the final product has gained a constant degree of interest. A strategy developed in the prior art is to use recombinant tropoelastin, or elastin-like peptides (ELP) produced by genetically modified organisms. Such peptides are then *in situ,* i.e. shortly after printing, crosslinked. For example, in Advanced Materials 32(45) (2020) 1-10, a recombinant tropoelastin functionalized with methacrylic groups combined with gelatin-methacrylate in photocrosslinkable bioinks for printing vascularized tissues with high cell-viability have been reported.

The disadvantage of recombinant tropoelastin and ELPs is that they are very expensive and obtainable only in low yields.

To overcome this problem, efforts were made to use elastin itself instead of said tropoelastin or ELPs. Elastin is available in large amounts i.e. in the meat production sector and therefore is not limited by the problems of tropoelastin and ELPs. In ACS omega 6(33) (2021) 21368-21383, elastin extracted from bovine neck ligament as a component of biomaterial inks has been used. In this work, elastin has been added in an amount of 1 wt% together with 10 wt% GelMA or 0.5 wt% collagen. Hence, due to the low solubility of the elastin in water, the elastin has been added in ground form, allowing only for a minor amount. Consequently, this application has the disadvantage of being limited in achieving the desired properties resulting from elastin.

Thus, there is the constant need of finding a 3D bioprinting process achieving products with properties comparable to properties resulting from the presence of elastin. Consequently, it is also an object to find biocompatible articles made from hydrogels, which exhibit properties such as caused by the presence of elastin in tissues. Thus, it is an object of the present invention to find such 3D bioprinting process and articles.

It has now surprisingly found out that this object can be achieved by a process for producing a biocompatible three-dimensional article, the process comprising the steps of

- providing in an elastin derivative provision step a water-soluble elastin derivative,

- producing in a hydrogel production step a hydrogel for 3D bioprinting comprising said water-soluble elastin derivative, and

- 3D bioprinting in a bioprinting step the biocompatible three-dimensional article by extrusion of said hydrogel.

Furthermore, it has been found out that this object can be achieved by the use of a hydrogel comprising a water-soluble elastin derivative for 3D bioprinting. It has been further found out that these objects can be achieved by the use of a hydrogel comprising a water-soluble elastin derivative for 3D bioprinting.

**Brief Description of the Figures**

Figure 1    shows structures printed from methacrylated α-elastin (ELMA)/GelMA biomaterial ink after photocuring. Figure 1A shows a rectangular gridded structure. Figure 1B shows tubular structures.

Figure 2    shows a tubular structure with a diameter of approx. 5 mm and a height of approximately 10 mm printed from κ-ELMA/GelMA (1:3) biomaterial ink after photocuring.

Figure 3    shows α-elastin release from printed structures. Particularly, Figure 3A shows the α-elastin concentration in the supernatant after printing (0 h) and after 2 h and 72 h of incubation in PBS, Figure 3B depicts SDS-PAGE analysis of supernatants from single (section 2) and double layer structures (section 3) after printing (3C) and after 2 h (3B) and 72 h (3A). References used are: α-elastin, 6 μg (3A), 3 μg (3B); molecular-weight size marker (L)

Figure 4    depicts vascular replicates printed from ELMA/GelMA (1:2) biomaterial ink. Figures 4A and 4B are replicates printed from a 3D data set that was generated from micro-CT scan of porcine aorta composed of photocured ELMA/GelMA biomaterial ink (1:2). 4C is a representation of the 3D model of porcine aorta with a large main vessel and a smaller off-branching vessel. Scale bars: 10 mm

Figure 5    shows a MTT test results from cultivated HuH7 cells printed within α-ELMA/GelMA (1:1) bioink. A negative control was printed without cells and cultured under the same conditions. No interfering signals were detected.

**Detailed Description of the Invention**

*Process of the invention*

In the following, the process of the present invention is described. Generally, the present invention is based on the finding that elastin can be brought into a soluble elastin derivative form, which can be used in hydrogels as bioink or biomaterial ink for 3D bioprinting. The elastin derivative achieves similar properties in the resulting three-dimensional biocompatible structure such as elastin in tissues. This is especially true as the soluble elastin derivative allows for significantly higher amounts in the hydrogel than native elastin.

The term *'biomaterial ink'* as used herein denotes cell-free hydrogels for 3D bioprinting applications and is used according to the nomenclature suggested in J Groll *et al* 2019 *Biofabrication* **11** 013001. In particular, but not exclusively, these are based on proteins, preferably but not limited to structural proteins, and more preferably but not limited to methacrylated gelatin (GelMA). As no cells are present in biomaterial inks, the presence of further components i.e. for providing a cytocompatible environment is not necessarily needed. Preferably, the solvent of the biomaterial ink is water.

The term *'bioink'* as used herein denotes cell-loaded hydrogels and is used according to the nomenclature suggested in J Groll *et al* 2019 *Biofabrication* **11** 013001. Hence, preferably, bioinks comprise at least one biomaterial ink and at least one cell, preferably a plurality of cells. Preferably, the solvent of the bioink is water. Also preferably, the bioink comprises further compounds for providing a cytocompatible environment such as nutrients, stabilizers etc. Especially, cell culture medium containing amino acids, vitamins, inorganic salts, glucose and serum is added. The medium provides nutrients, ensures the maintenance of osmolarity and stabilizes the pH value.

The term *'3D bioprinting'* as used herein denotes extrusion-based 3D bioprinting, i.e. based on the ejection of a material through a nozzle, usually mobile in three directions, onto a platform.

The term *'elastin'* as used herein denotes a biopolymer present in the extracellular matrix of vertebrates providing elasticity and resilience to tissues and organs. Elastin has outstanding characteristics such as a very low elastic modulus, a very high biological half-life and a high resistance to proteolytic enzymes. Elastin is isolated from vertebrate tissues (e. g. aorta, ligaments) and is insoluble in all organic and inorganic solvents. As used herein the term *'native elastin'* denotes elastin as extracted from the extracellular matrix without any further modification.

Elastin can be obtained in large amounts as a by-product of meat production. However, this elastin is insoluble in water and thus needs to be modified to be soluble. Generally, any method allowing for a reduction of the protein chain length in the elastin at maintenance of elastin-specific amino acid sequence motifs in the resulting protein fragments to achieve properties similar to those of native elastin can be used to achieve water-soluble elastin derivatives. Such amino acid sequence motifs can be, but are not limited to, VPGVG, GVPGV, PGVGV, GVPGVG, PGVGVA, GVPG, GXPG, VPGXG, GXVPG, GXXPG, GXVPGX where X can represent all amino acids except P.

Preferably, however, the elastin derivative provision step of the inventive process of the present invention comprises, preferably consists of, hydrolyzing elastin. Hence, preferably, the water-soluble elastin derivative comprised in the hydrogel is hydrolyzed elastin. In such a reaction, native elastin is hydrolyzed using chemical and/or enzymatic treatment thereby producing fragments of the elastin protein having different sizes. Hence, preferably, the elastin derivative provision step of the inventive process of the present invention comprises, preferably consists of, hydrolyzing the elastin by chemical, preferably acidic or basic, or enzymatic hydrolysis.

The degree of hydrolyzation can be varied by the incubation time or the type and efficiency of the hydrolyzation agent. According to the hydrolyzation method, the distribution of the molecular weight of the resulting elastin derivative includes fragments with a molecular weight from as low as 1 kDa up to over 700 kDa. Besides controlling hydrolysis, the molecular weight distribution of elastin derivative can be defined by fractionation (e.g., membrane filtration with different molecular weight cut-off values) or by using more than one type of elastin hydrolysate. The molecular weight distribution of the elastin derivative and the overall ratio of the weight of the elastin derivative to the weight of the dry mass of the hydrogel polymer are parameters to tailor the physicochemical and mechanical properties of the hydrogels and hence the printed structures. Particularly, partial acidic hydrolysis leads to high molecular weight fragments, wherein the product of the produced elastin derivative is usually called $\alpha$-elastin. $\alpha$-elastin retains most of the physicochemical properties and the amino acid composition of elastin. On the other hand, a fractionation process of $\alpha$-elastin leads to a fraction with low molecular weight fragments, to which it is referred to as $\beta$-elastin. Alternatively, soluble elastin with low mean molecular weight can be obtained by basic hydrolysis, the product of which is referred to as $\kappa$-elastin.

Hence, more preferably, the elastin derivative provision step of the inventive process of the present invention comprises, preferably consists of, hydrolyzing the elastin by acidic or basic hydrolysis, most preferably by acidic hydrolysis. Consequently, more preferably, the elastin derivative of the hydrogel is selected from the list consisting of $\alpha$-elastin, $\beta$-elastin, $\kappa$-elastin, or mixtures thereof. Due to the closest resembling of the properties of elastin, the elastin derivative of the hydrogel most preferably is $\alpha$-elastin.

Preferably, the elastin used in the process of the present invention is extracted from animal tissues, more preferably vertebrate tissues, and most preferably mammalian tissues. Preferable mammalian tissues are selected from the list consisting of bovine, porcine, equine, murine, sheep, chicken, rat and rabbit tissues as well as marine vertebrate tissues. In special cases, donor tissues from human might also be considered.

Elastin peptides can be released from bioprinted structures by chemical or enzymatical hydrolysis. By means of mass spectrometry, elastin peptides originating from extracted elastin according to the claims of the current invention can be identified by the presence of hydroxyproline. This is a natural modification of proline residues that is only present in specific amino acid sequence motifs of extracted elastin, but absent in biotechnologically produced elastin-like materials (tropoelastin, elastin-like peptides, etc.). Hence, preferably, the water-soluble elastin derivative of hydrogel used in the process of the present invention comprises hydroxyproline. Alternatively and preferably, the elastin derivative of the present invention comprises specific cross-linking amino acids, such as first and foremost desmosine, isodesmosine, merodesmosine and lysinonorleucine. These amino acids can be detected by the method of amino acid analysis.

To achieve properties closely resembling those induced by the elastin e.g. in mammal tissue, certain amounts of water-soluble elastin derivative needs to be present in the hydrogel as used in the process of the present invention. Hence, preferably, the water-soluble elastin derivative is present in the hydrogel as used in the process of the present invention in an amount of at least 5 wt% with respect to the total weight of the dry mass of the hydrogel polymer, more preferably in the range of 10 wt% to 80 wt%, even more preferably in the range of 30 wt% to 50 wt%, and most preferably at 30 wt%.

Such as elastin is also accompanied by many other proteins in mammal tissue resulting in a specific property profile, also the properties of the three-dimensional biocompatible structure can be tailored. The interplay of elastin and collagen in connective tissues is crucial for the overall mechanical properties of organs and tissues. Collagen imparts tensile strength while elastin enables tissues to stretch and recoil. Currently, most protein-based inks for bioprinting are based on methylacrylated gelatin (GeIMA). The introduction of water-soluble elastin derivative to bioprinting enables the fine-tuning of the mechanical properties of hydrogels at minimal costs and efforts when compared to previous technical solutions. Moreover, the water-soluble elastin derivative adds additional biochemical cues to the biomaterial matrix, which supports cell growth and cultivation.

The term 'gelatin' as used herein denotes the irreversibly hydrolyzed form of collagen, wherein the hydrolysis reduces protein fibrils into smaller peptides. Gelatin as used in the examples herein is IMAGEL provided by Gelita AG, Germany or EMPROVE ESSENTIAL Gelatin powder provided by Merck AG.

The term 'collagen' as used herein denotes the main structural protein in the extracellular matrix found tissues of mammals. As such, it is the most abundant protein in mammals, making up from 25% to 35% of the whole-body protein content. Collagen consists of amino acids bound together to form a triple helix of elongated fibril known as a collagen helix.

Hence, preferably, in the process according to the present invention, the hydrogel further comprises at least one second polymer, wherein the at least one second polymer preferably comprises a polymer of biological origin, a polymer of non-biological origin, or mixtures thereof. Preferably, the polymer of biological origin is selected from the list consisting of polypeptides, polysaccharides, or mixtures thereof, more preferably the polymer of biological origin is selected from the list consisting of gelatin, collagen, and mixtures thereof, and most preferably the polymer of biological origin is gelatin. Preferably, the polymer of non-biological origin is a biocompatible polymer of non-biological origin, more preferably is a nonionic polyoxyethylene-polyoxypropylene block copolymer.

Hence, the hydrogel production step comprises, preferably consists of, the step of mixing the elastin derivative and optionally the second polymer with a solvent. Usually, the solvent used in the hydrogel production step of the process of the present invention comprises, preferably consists of, water. Furthermore, the hydrogel of the present invention should be biocompatible and cytocompatible to be usable for 3D bioprinting. The hydrogel preferably has a high viscous state at a temperature below 12 °C, more preferably, below 18 °C, and most preferably below room temperature.

Preferably, the weight ratio between the weight of the elastin derivative to the weight of the second polymer in the hydrogel is in the range of 10:1 to 1:10, more preferably 3:1 to 1:3, even more preferably 3:2 to 2:3, and most preferably is 1:1.

The term 'biocompatible' as used herein denotes the ability of a material to perform with an appropriate host response in a specific application.

The term 'cytocompatible' as used herein denotes a substance or material not having any adverse effect on cells in culture.

Typically, the articles made by 3D bioprinting are intended to be used in or at the human body. As such, they need different grades of structural resistance. Such resistance can be enhanced by various methods.

Generally, such stabilization can be achieved by means of physical, chemical, photochemical, enzymatic, or thermal treatment resulting in non-covalent interactions or covalent crosslinking among elastin polypeptide chains or between elastin polypeptide chains with at least one other component of the hydrogel.

Hence, in certain cases, the hydrogels are cured after 3D bioprinting to stabilize the resulting printed structures. Curing is also known as crosslinking, wherein covalent bonds are formed between the protein carbon chains fixing their positions. Thereby, generally, any known biocompatible type of crosslinking can be used.

The process of the present invention therefore preferably comprises after the bioprinting step crosslinking in a crosslinking step at least a part of the water-soluble elastin derivative, the second polymer, or both.

One way to achieve crosslinking, especially without any further modification of the used protein polymers, is bringing the three-dimensional biocompatible structure in contact with formaldehyde, preferably in a formaldehyde atmosphere. Under such conditions, the formaldehyde reacts with N-terminal amino groups and hydroxyphenyl groups of side chains of *inter alia* tyrosine at the protein backbone thereby forming covalent bonding bridges between side chains of the backbone carbon chains of the protein(s).

In a different approach, modified water-soluble elastin derivative in the hydrogel is used in the process of the present invention. Such modification is introduced to the water-soluble elastin derivative by means of enzymatic or most preferably chemical treatment of at least one reactive site of the water-soluble elastin derivative. Preferably, said reactive site is a functional group of the water-soluble elastin derivative, preferably a functional group selected from the list consisting of primary amines ($-NH_2$), secondary amines (-NH-), carbonyl groups (-C=O), hydroxyl groups (-OH), carboxyl groups (-COOH), thiol groups (-SH), phenyl groups ($-C_6H_5$), hydroxyphenyl groups ($-C_6H_5O$) or guanidine groups ($-CH_4N_3$). Such modified functional groups can then serve as active sites for crosslinking agents. Preferably, the modified functional groups are groups having terminal C-C double bonds, most preferably methacryloyl groups. More preferably, the modifications enable the material to be crosslinked by photoactivation which requires the addition of a photoinitiator. Such coupling strategy for photocuring relies on radical polymerization of methacrylated amino acid side chains. This requires a photoinitiator, such as lithium phenyl(2,4,6-trimethylbenzoyl)phosphinate (LAP). Methacrylated proteins can also be crosslinked by homobifunctional or homomultifunctional thiol crosslinking agents. Photoactivated crosslinking has many advantages. One of the major advantages is that the curing time usually is fast. Another advantage is that no mechanical interaction with the three-dimensional biocompatible structure is needed.

Thus, preferably the process of the present invention comprises after the elastin derivative provision step the step of modifying in an elastin derivative modifying step the water-soluble elastin derivative. More preferably, the elastin derivative modifying step comprises the step of modifying at least one functional group of the water-soluble elastin derivative yielding a modified functional group, wherein, preferably, the functional group is selected from the list consisting of primary amines ($-NH_2$), secondary amines (-NH-), carbonyl groups (-C=O), hydroxyl groups (-OH), carboxyl groups (-COOH), thiol groups (-SH), phenyl groups ($-C_6H_5$), hydroxyphenyl groups ($-C_6H_5O$) or guanidine groups ($-CH_4N_3$). Preferably, the modified functional group is a group having terminal C-C double bonds, most preferably a methacryloyl group.

In an alternative preferred embodiment, the modified functional group is a group known from the technical field of photosensitive click chemistry. Hence, preferably the modified functional group is a functional group having C-C triple bonds and/or azide groups. In such an embodiment a crosslinking agent is not needed, as the coupling reaction (azide-alkine cycloaddition) can directly be activated by light irradiation.

Preferably, the crosslinking step comprises the step of coupling the modified functional group, wherein the functional group is a group having terminal C-C double bonds, most preferably a methacryloyl group, to primary amines and hydroxyl groups of amino acid side chains of polypeptide chains of the elastin derivative.

Also preferably, the crosslinking step of the process of the present invention comprises the step of adding a crosslinking agent. Preferably, the crosslinking agent can be selected from a chemical crosslinking agent or a photoinitiator. Preferred embodiments of the chemical crosslinking agent are selected from formaldehyde, homobifunctional thiol crosslinking agents or homomultifunctional thiol crosslinking agents. While homobifunctional thiol crosslinking agents or homomultifunctional thiol crosslinking agents can crosslink with cysteine groups, in absence of such groups the modification yielding a methacryloyl group as described above is necessary for crosslinking to occur. If the crosslinking agent is a photoinitiator, subsequently to the step of adding the photoinitiator, the step of crosslinking comprises the step of photo-activating said photoinitiator. Preferably, the photoinitiator is selected from the list consisting of lithium phenyl(2,4,6-trimethylbenzoyl)phosphinate (LAP), 2-(2,4,5,7-tetrabromo-6-oxido-3-oxo-3H-xanthen-9-yl)benzoate, 2-Hydroxy-1-[4-(2-hydroxyethoxy)phenyl]-2-methyl-1-propanone, 2-hydroxy-2-methylpropiophenone, or mixtures thereof, most preferably the photoinitiator is lithium phenyl(2,4,6-trimethylbenzoyl)phosphinate (LAP). Most preferably, the photoinitiator is 0.25 wt% lithium phenyl(2,4,6-trimethylbenzoyl)phosphinate (LAP). Preferably, the crosslinking agent is added in an amount in the range of 0.1 wt% to 1 wt% with respect to the weight of the dry mass of the hydrogel polymer. Preferably, the step of activating the photoinitiator comprises, preferably consists of, irradiating the photoinitiator with light.

Hence, an especially preferred embodiment of the present invention relates to a process for producing a biocompatible three-dimensional article, the process comprising the steps of

- providing in an elastin derivative provision step a water-soluble elastin derivative, wherein the water-soluble elastin derivative is preferably selected from the list consisting of a hydrolyzed elastin, preferably α-elastin, β-elastin, κ-elastin, or mixtures thereof,

- producing in a hydrogel production step a hydrogel for 3D bioprinting comprising said water-soluble elastin derivative, wherein the hydrogel further comprises at least one second polymer, wherein the at least one second polymer preferably comprises a polymer of biological origin, a polymer of non-biological origin, or mixtures thereof,

- 3D bioprinting in a bioprinting step the biocompatible three-dimensional article by extrusion of said hydrogel,

- crosslinking in a crosslinking step at least a part of the water-soluble elastin derivative, the at least one second polymer, or both, wherein the crosslinking step preferably comprises the step of contacting the three-dimensional article with a crosslinking agent, preferably formaldehyde.

In a further especially preferred embodiment of the present invention relates to a process for producing a biocompatible three-dimensional article, the process comprising the steps of

- providing in an elastin derivative provision step a water-soluble elastin derivative, wherein the water-soluble elastin derivative is preferably selected from the list consisting of a hydrolyzed elastin, preferably $\alpha$-elastin, $\beta$-elastin, $\kappa$-elastin, or mixtures thereof,

- modifying in an elastin derivative modifying step said water-soluble elastin derivative yielding a modified water-soluble elastin derivative, wherein, preferably, the elastin derivative modifying step comprises the step of modifying at least one functional group of the water-soluble elastin derivative yielding a modified functional group, wherein, preferably, the functional group is selected from the list consisting of primary amines ($-NH_2$), secondary amines (-NH-), carbonyl groups (-C=O), hydroxyl groups (-OH), carboxyl groups (-COOH), thiol groups (-SH), phenyl groups ($-C_6H_5$), hydroxyphenyl groups ($-C_6H_5O$) or guanidine groups ($-CH_4N_3$).

- producing in a hydrogel production step a hydrogel for 3D bioprinting comprising said modified water-soluble elastin derivative,

- 3D bioprinting in a bioprinting step the biocompatible three-dimensional article by extrusion of said hydrogel,

- crosslinking in a crosslinking step at least a part of the modified water-soluble elastin derivative, wherein the crosslinking step preferably comprises the step of adding a photoinitiator and subsequently photo-activating said photoinitiator, wherein, preferably, the photoinitiator is selected from the list consisting of lithium phenyl(2,4,6-trimethylbenzoyl)phosphinate (LAP), 2-(2,4,5,7-tetrabromo-6-oxido-3-oxo-3H-xanthen-9-yl)benzoate, 2-Hydroxy-1-[4-(2-hydroxyethoxy)phenyl]-2-methyl-1-propanone, 2-hydroxy-2-methylpropiophenone, or mixtures thereof, most preferably the photoinitiator is lithium phenyl(2,4,6-trimethylbenzoyl)phosphinate (LAP).

Moreover, another especially preferred embodiment of the present invention relates to a process for producing a biocompatible three-dimensional article, the process comprising the steps of

- providing in an elastin derivative provision step a water-soluble elastin derivative, wherein the waterssoluble elastin derivative is preferably selected from the list consisting of a hydrolyzed elastin, preferably $\alpha$-elastin, $\beta$-elastin, $\kappa$-elastin, or mixtures thereof,

- modifying in an elastin derivative modifying step said water-soluble elastin derivative yielding a modified water-soluble elastin derivative, wherein, preferably, the elastin derivative modifying step comprises the step of modifying at least one functional group of the water-soluble elastin derivative yielding a modified functional group, wherein, preferably, the functional group is selected from the list consisting of primary amines ($-NH_2$), secondary amines (-NH-), carbonyl groups (-C=O), hydroxyl groups (-OH), carboxyl groups (-COOH), thiol groups (-SH), phenyl groups ($-C_6H_5$), hydroxyphenyl groups ($-C_6H_5O$) or guanidine groups ($-CH_4N_3$).

- producing in a hydrogel production step a hydrogel for 3D bioprinting comprising said modified water-soluble elastin derivative, wherein the hydrogel further comprises at least one second polymer, wherein the at least one second polymer preferably comprises a polymer of biological origin, a polymer of non-biological origin, or mixtures thereof

- 3D bioprinting in a bioprinting step the biocompatible three-dimensional article by extrusion of said hydrogel,

- crosslinking in a crosslinking step at least a part of the modified water-soluble elastin derivative, the at least one second polymer, or both, wherein the crosslinking step preferably comprises the step of adding a photoinitiator and subsequently photo-activating said photoinitiator, wherein, preferably, the photoinitiator is selected from the list consisting of lithium phenyl(2,4,6-trimethylbenzoyl)phosphinate (LAP), 2-(2,4,5,7-tetrabromo-6-oxido-3-oxo-3H-xanthen-9-yl)benzoate, 2-Hydroxy-1-[4-(2-hydroxyethoxy)phenyl]-2-methyl-1-propanone, 2-hydroxy-2-methylpropiophenone, or mixtures thereof, most preferably the photoinitiator is lithium phenyl(2,4,6-trimethylbenzoyl)phosphinate (LAP).

*Article of the invention*

The present invention relates to an article comprising a hydrogel comprising a water-soluble elastin derivative. In particular, the article is a biocompatible and a cytocompatible article. Preferably, the article can be used to be implanted in or at the human body. Hence, examples of articles may be vascular tissues and objects made thereof. Hence, such article may even be a whole organ. It is preferred that the hydrogel used for printing the article comprises cells.

The article comprises the hydrogel as used in the 3D bioprinting process. In case no crosslinking has been carried out, the formulation of the article is identical to the formulation of the hydrogel. However, due to evaporation or hygroscopic issues, the water content may vary. The article may be further processed. In particular, the cells in the article might be chosen in a way to further grow or otherwise transform the hydrogel and its ingredient so that the formulation of the article is modified.

Preferably, the elastin derivative comprised in the article of the present invention is hydrolyzed elastin. More preferably, the elastin derivative of the article of the present invention is selected from the list consisting of α-elastin, β-elastin, κ-elastin, or mixtures thereof. Due to the closest resembling of the properties of elastin, the elastin derivative of the article of the present invention most preferably is α-elastin.

Preferably, the elastin used in the article of the present invention is extracted from animal tissues, more preferably vertebrate tissues, and most preferably mammalian tissues. Preferable mammalian tissues are selected from the list consisting of bovine, porcine, equine, murine, sheep, chicken, rat and rabbit tissues as well as marine vertebrate tissues. In special cases, donor tissues from human might also be considered.

To achieve properties closely resembling those induced by the elastin e.g. in mammal tissue, certain amounts of water-soluble elastin derivative needs to be present in the article of the present invention. Hence, preferably, the water-soluble elastin derivative is present in the article of the present invention in an amount of at least 5 wt% with respect to the total dry weight of the hydrogel, more preferably in the range of 10 wt% to 80 wt%, even more preferably in the range of 30 wt% to 50 wt%, and most preferably at 30 wt%.

Hence, preferably, the article of the present invention further comprises at least one second polymer, wherein the at least one second polymer preferably comprises a polymer of biological origin, a polymer of non-biological origin, or mixtures thereof. Preferably, the polymer of biological origin is selected from the list consisting of polypeptides, polysaccharides, or mixtures thereof, more preferably the polymer of biological origin is selected from the list consisting of gelatin, collagen, and mixtures thereof, and most preferably the polymer of biological origin is gelatin. Preferably, the polymer of non-biological origin is a biocompatible polymer of non-biological origin, more preferably is a nonionic polyoxyethylene-polyoxypropylene block co-polymer.

Preferably, the weight ratio between the weight of the elastin derivative to the weight of the second polymer in the article of the present invention is in the range of 10:1 to 1:10, more preferably 3:1 to 1:3, even more preferably 3:2 to 2:3, and most preferably is 1:1. Usually, the solvent used in the article of the present invention comprises, preferably consists of water.

Preferably, the article of the present invention is crosslinked, i.e., the water-soluble elastin derivative, the at least second polymer, or both, have been crosslinked by a crosslinking agent and/or photo-activation as explained for the process of the present invention above.

*Use of the invention*

The present invention relates to an use of a hydrogel comprising a water-soluble elastin derivative for 3D bioprinting a biocompatible three-dimensional article. In particular, the hydrogel is biocompatible and cytocompatible.

Preferably, the elastin derivative comprised in the hydrogel as used in the present invention is hydrolyzed elastin. More preferably, the elastin derivative of the hydrogel as used in the present invention is selected from the list consisting of α-elastin, β-elastin, κ-elastin, or mixtures thereof. Due to the closest resembling of the properties of elastin, the elastin derivative of the hydrogel as used in the present invention most preferably is α-elastin.

Preferably, the elastin in the hydrogel used in the present invention is extracted from animal tissues, more preferably vertebrate tissues, and most preferably mammalian tissues. Preferable mammalian tissues are selected from the list consisting of bovine, porcine, equine, murine, sheep, chicken, rat and rabbit tissues as well as marine vertebrate tissues. In special cases, donor tissues from human might also be considered.

To achieve properties closely resembling those induced by the elastin e.g., in mammal tissue, certain amounts of water-soluble elastin derivative needs to be present in the hydrogel used in the present invention. Hence, preferably, the water-soluble elastin derivative is present in the hydrogel used in the present invention in an amount of at least 5 wt% with respect to the total dry weight of the hydrogel, more preferably in the range of 10 wt% to 80 wt%, even more preferably in the range of 30 wt% to 50 wt%, and most preferably at 30 wt%.

Hence, preferably, the hydrogel used in the present invention further comprises at least one second polymer, wherein the at least one second polymer preferably comprises a polymer of biological origin, a polymer of non-biological origin, or mixtures thereof. Preferably, the polymer of biological origin is selected from the list consisting of polypeptides, polysaccharides, or mixtures thereof, more preferably the polymer of biological origin is selected from the list consisting of gelatin, collagen, and mixtures thereof, and most preferably the polymer of biological origin is gelatin. Preferably, the polymer of non-biological origin is a biocompatible polymer of non-biological origin, more preferably is a nonionic polyoxyethylene-polyoxypropylene block co-polymer.

Preferably, the weight ratio between the weight of the elastin derivative to the weight of the second polymer in the hydrogel used in the present invention is in the range of 10:1 to 1:10, more preferably 3:1 to 1:3, even more preferably 3:2 to 2:3, and most preferably is 1:1. Usually, the solvent in the hydrogel used in the present invention comprises, preferably consists of water.

Preferably, the hydrogel used in the present invention can be crosslinked, i.e. the water-soluble elastin derivative, the at least second polymer, or both, can be crosslinked by a crosslinking agent and/or photo-activation as explained for the process of the present invention above.

## Examples

*Abbreviations*

The following abbreviations for compounds and solutions are used in the description and the examples.

| | |
|---|---|
| CB | Carbonate-bicarbonate buffer (0.25 M, pH 9.2) |
| $ddH_2O$ | Double distilled water |
| DMEM | Dulbecco's Modified Eagle Medium |
| DPBS | Dulbecco's Phosphate Buffered Saline, pH 7.4 |
| ELMA | Methacrylated soluble elastin derivative (i.e. $\alpha$-elastin, $\beta$-elastin, $\kappa$-elastin) |
| F127 | Pluronic F-127 (Sigma-Aldrich, mean molecular weight 12.6 kDa, P2443), which is a nonionic polyoxyethylene-polyoxypropylene block co-polymer with the general formula $HO(C_2H_4O)_a(-C_3H_6O)_b(C_2H_4O)_aH$. |
| F127-DMA | Pluronic F-127 dimethacrylate |
| GelMA | Methacrylated gelatin |
| LAP | Lithium phenyl(2,4,6-trimethylbenzoyl)phosphinate (provided by CELLINK AB, Gothenburg, Sweden) |
| MA | Methacrylic anhydride |
| MTT | 3-(4,5-dimethylthiazol-2-yl)-2,5-diphenyltetrazolium bromide |
| PBS | Phosphate-buffered saline, pH 7.4 |

| | |
|---|---|
| SDS | Sodium dodecyl sulfate |

*Measurement methods*

a) *Degree of modification of primary amino groups*

The degree of modification of primary amino groups in $\alpha$-ELMA was determined by fluorescence spectroscopy using the o-phthalaldehyde (OPA) assay (Fluoraldehyde Reagent Solution, Thermo Fisher) according to the supplier's protocol. Briefly, sample solutions and OPA reagent were equilibrated to room temperature and 200 $\mu$L of OPA reagent solution was subsequently added to 20 $\mu$L of sample (500 $\mu$g/mL) in a 96-well plate. Samples were analyzed with an excitation wavelength of 340 nm and fluorescence emission was detected at 460 nm using a FLUOstar OPTIMA microplate reader (BMG Labtech). As a reference, non-modified $\alpha$-elastin was used. The degree of modification $\Phi$ is calculated according to equation 1, where $I$ is the absorbance. Double distilled water (ddH$_2$O) was used as blank.

$$\phi = 1 - \frac{I_{sample} - I_{blank}}{I_{reference} - I_{blank}} \qquad \text{Equation 1}$$

b) *Concentration of $\alpha$-elastin peptides release*

analyzed by SDS-PAGE (10% separation gel, 100 $\mu$L sample volume) and Bradford assay (50 $\mu$L sample volume) using $\alpha$-elastin as reference

c) *Determination of the cell number*

The cell number was determined by MTT assay. Samples were rinsed with PBS and the hydrogels were transferred to a sterile Eppendorf tube and the weight of the samples was determined after removal of excess liquid (centrifugation). Afterwards, 350 $\mu$L MTT solution composed of 10% MTT stock solution (5 mg/mL MTT in PBS, sterile) and 90% completed DMEM was added to each tube. The samples were subsequently incubated at 37 °C, 8% CO$_2$ and rH >95% for 3.5 h. The samples were then centrifuged, and the supernatant was removed before 400 $\mu$L extraction solution (9 parts isopropanol, 1 part 10% SDS solution, 0.004 parts 32% HCl) was added to each tube. Samples were incubated on a shaker (300 rpm) at room temperature for 1 h protected from light. The supernatant was removed after centrifugation at 14,100 g for 3 min. The pellets were subsequently extracted a second time with 400 mL DMSO and 4 mM HCl and disintegrated using an ultrasonic disperser. The extinction of the supernatants was measured at 570 nm and 720 nm (reference wavelength). OD values were normalized to 100 mg of sample.

*Example 1: Preparation of methacrylated α-elastin (a-ELMA)*

Elastin was isolated from porcine aorta and hydrolyzed with oxalic acid as disclosed in Example 2 of EP 3 581 212 A1 (paragraphs [0042] - [0043]). Further on, lyophilized α-elastin was added to cold PBS (4 °C) at a concentration of 10% (w/v) and stirred at 4 °C until a solution was obtained. Subsequently, MA was added at a rate of 0.5 mL/min to a final concentration of 8% (v/v). The solution was stirred at 4 °C for 7 h before it was 3x diluted with cold PBS. The produced α-ELMA was then dialyzed against distilled water at 4 °C using pre-wetted regenerated cellulose dialysis tubes with a 1 kDa molecular weight cut-off. α-ELMA powder was afterwards isolated by lyophilization. For the produced α-ELMA the degree of modification of primary amino groups was $\Phi=0.77$.

This example shows that α-elastin can be modified with photocrosslinkable chemical entities in a sufficiently high degree of modification. Similar methods as they are known in the art for the preparation of GelMA can be applied for this purpose. Crucial to this method is the temperature of 4 °C to prevent elastin aggregation.

*Example 2: Preparation of methacrylated κ-elastin (κ-ELMA)*

Elastin was isolated from porcine aorta and hydrolyzed with potassium hydroxide as disclosed in Example 1 in EP 3 581 212 A1 (paragraphs [0038] and [0039]). Lyophilized κ-elastin was dissolved in PBS at 4°C and modified with MA as described in Example 1. The produced κ-ELMA was dialyzed and subsequently isolated by lyophilization. The degree of modification of primary amino groups in the produced κ-ELMA was $\Phi=0.80$.

This example shows that photocrosslinkable κ-elastin can be prepared by similar methods as they are known in the art for the preparation of GelMA with a sufficiently high degree of modification. Crucial to this method is the temperature of 4 °C to prevent elastin aggregation.

*Example 3: Preparation of methacrylated gelatin (Gel-MA)*

This example repeats knowledge from the prior art. GelMA was prepared according to a protocol published by Yu et al. (Paragraph 2.2 in Yue K, Li X, Schrobback K, Sheikhi A, Annabi N, Leijten J, Zhang W, Zhang YS, Hutmacher DW, Klein TJ, Khademhosseini A. Structural analysis of photocrosslinkable methacryloyl-modified protein derivatives. Biomaterials. 2017 Sep;139:163-171. doi: 10.1016/j.biomaterials.2017.04.050. Epub 2017 May 29. PMID: 28618346; PMCID: PMC5845859.). In brief, gelatin (EMPROVE exp, Ph. Eur., Merck) was dissolved in pre-heated PBS (50-55 °C) at 10% (w/v). MA was added to a final concentration of 8% (v/v) and stirred for 1 h. Afterwards, the solution was diluted by adding 2 volumes of heated PBS. The GelMA solution was dialyzed against distilled water using pre-wetted regenerated cellulose dialysis tubes with a 1 kDa molecular weight cut-off. GelMA powder was subsequently extracted by lyophilization.

*Example 4: Preparation of α-ELMA/GelMA biomaterial ink*

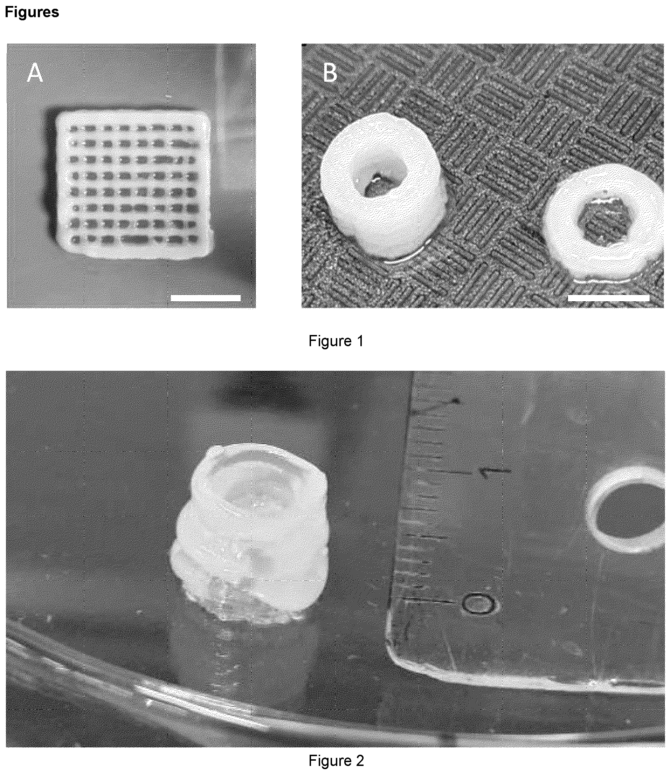

GelMA prepared according to Example 3 was added to pre-heated PBS (50 °C) at 5% (w/v) and stirred until the material dissolved completely. After the solution was cooled to room temperature, 5% α-ELMA produced according to Example 1 was added and stirred to homogeneity. Prior to printing, 0.25% (w/v) to LAP was added, and the preparation was stirred again. The ink preparation was transferred to tainted cartridges and refrigerated for proper gelation. For printing flat grid-like structures (Figure 1A), the temperature-controlled printhead was set to 21 °C and the printbed temperature to 10 °C. Pressure and printing speed were adjusted between 13 kPa and 18 kPa and between 7 mm/s and 10 mm/s, respectively. Pre- and post-printing times were set to 20 ms.

Flat structures (Figure 1A) and hollow cylinders with a diameter of 5 mm and a height of approximately 1 cm (Figure 1B) were printed using blunt metal needles (22G, 0.5 inch, CELLINK AB, Gothenburg, Sweden) as a nozzle together with a metal heat cap on the temperature-controlled printhead. The printbed temperature was adjusted in the range of 11 °C to 14 °C and the printhead temperature was adjusted in the range of 18 °C and 21 °C, respectively. Extrusion pressure was varied between 45 kPa and 60 kPa and print speed was set to 9 mm/s and 12 mm/s.

Layer height was set to 0.27 mm and pre- and postprinting times were set to 4 ms and 30 ms, respectively. Photo-crosslinking was done every two layers at 405 nm for 30 s using the photocuring toolhead provided with the bioprinter.

This example shows that α-ELMA can be implemented in a GelMA hydrogel in various ratios, e.g., a 1:1 ratio with respect to the total protein dry mass as described herein. Upon photocrosslinking, stable structures are formed. Photocrosslinked α-ELMA/GelMAhydrogels are suitable for printing both flat and high geometric structures.

*Example 5: Preparation of κ-ELMA/GelMA composite bioink*

GelMA prepared according to Example 3 was added to pre-heated PBS (approx. 50 °C) at 7.5% (w/v) and stirred until the material dissolved completely. After the solution was cooled to room temperature, 2.5% κ-ELMA produced according to Example 2 was added and stirred to homogeneity. Prior to printing, 0.25% (w/v) LAP was added, and the preparation was stirred again. The ink preparation was transferred to tainted cartridges and refrigerated for proper gelation.

Hollow cylinders with a diameter of 5 mm and a height of approximately 1 cm (Figure 2) were printed using blunt metal needles (22G, 0.5 inch, provided by CELLINK AB, Gothenburg, Sweden) as a nozzle together with a metal heat cap on the temperature-controlled printhead. The printbed temperature was adjusted in the range of 11 °C to 14 °C and the printhead temperature was adjusted in the range of 18 °C and 21 °C, respectively. Extrusion pressure and print speed were varied in the ranges of 30 kPa to 45 kPa and 9 mm/s to 12 mm/s, respectively. Layer height was set to 0.23 mm and pre- and postprinting times were adjusted in the range of 5 ms to 7 ms and 15 ms to 35 ms, respectively. Each layer was photo-crosslinked at 405 nm for 40 s.

This example shows that κ-ELMA can be implemented in a GelMA hydrogel in various ratios, e.g., a 1:3 ratio with respect to the total protein dry mass as described herein. Upon photocrosslinking, stable structures are formed. Photocrosslinked κ-ELMA/GelMA hydrogels are suitable for printing both flat and high geometric structures.

*Example 6: Preparation of κ-ELMA/Pluronic dimethacrylate composite biomaterial ink*

1 g κ-elastin produced according to Example 2 was dissolved in 10 mL DPBS at 4 °C and 1.5 mL MA was added dropwise under continuous stirring. After 2 h, stirring was interrupted and the solution was refrigerated for 16 h. Afterwards, 10 mL of pre-cooled DPBS was added to stop the reaction.

In an alternative approach, 1 g κ-elastin was dissolved in CB at 4 °C. Subsequently, 50 μL MA was added dropwise under vigorous stirring and pH was adjusted to 9 with concentrated NaOH solution. This step was repeated every 20 min until 300 μL MA were added in total. The solution was then stirred for another 4 h until 10 mL CB were added and pH was lowered to 7 to quench the reaction.

For both approaches, κ-ELMA solution was dialyzed against deionized water for 7 days at 4 °C using pre-wetted regenerated cellulose dialysis tubes with a 1 kDa molecular weight cut-off. The product was subsequently lyophilized for approximately 24 h.

F-127 was functionalized by adapting the protocol of van Hove et al. (A.H. Van Hove, B.D. Wilson, D.S. Benoit, Microwave-assisted functionalization of poly(ethylene glycol) and on-resin peptides for use in chain polymerizations and hydrogel formation, J Vis Exp (80) (2013) e50890, p. 5). Briefly, 5 g of F-127 and 1.23 g MA (equivalent to 10:1 molar ratio relative to the hydroxyl content) were mixed in a glass vial and heated in a conventional microwave oven in 10 cycles of 30 s each at 500 W power alternated by 30 s vortex stirring. Subsequently, 10 mL tetrahydrofuran was added to the vial and the modified polymer was precipitated in 60 mL n-hexane, vacuum filtered and recovered on quantitative filter paper. The purification was repeated five times. For final drying, the produced F127-DMA was kept under vacuum for 24 h.

Biomaterial inks were prepared as hydrogels composed of

(1) 7.5 wt% κ-elastin and 22.5 wt% F127

(2) 7.5 wt% κ-ELMA, 22.5 wt% F127-DMA and 0.25 wt% LAP.

Biomaterial inks were transferred to 3 mL amber cartridges and printed on a 3D bioprinter (BIO X, provided by CELLINK AB, Gothenburg, Sweden). Prior to printing, the ink was maintained at a temperature of 25 °C for 30 min using the temperature-controlled printhead. Pre-fixed pressure parameters (75, 100, 125, 150, 175, and 200 kPa), printing speeds (3 mm/s - 8 mm/s) and nozzle diameters (22, 25, and 27G) were tested. Best printing results were achieved with a 22G nozzle, an extrusion pressure of 175 kPa and a printing speed of 6 mm/s.

It could be shown that both, methacrylated and pristine soluble elastin, can be implemented in a significant amount to hydrogels comprising polymers of non-biological origin. By modifying said polymer of non-biological origin with photocrosslinkable chemical entities, a stable network of ELMA and said photocrosslinkable polymer of non-biological origin is formed.

*Example 7: Preparation of biomaterial inks with elastin release*

Gelatin was completely dissolved in ddH$_2$O at approximately 60 °C. α-elastin was added under stirring after the solution cooled to 40 °C. The following concentrations were tested:

(1) 5 wt% gelatin and 7.5 wt% α-elastin

(2) 7.5 wt% gelatin and 7.5 wt% α-elastin

(3) 10 wt% gelatin and 10 wt% α-elastin

Best printing performance, uniformity and stability was achieved with formulation (2). Printed structures were optionally crosslinked by incubation in a formaldehyde-rich atmosphere inside a desiccator for 60 min above a reservoir of 20 mL 37% formaldehyde solution.

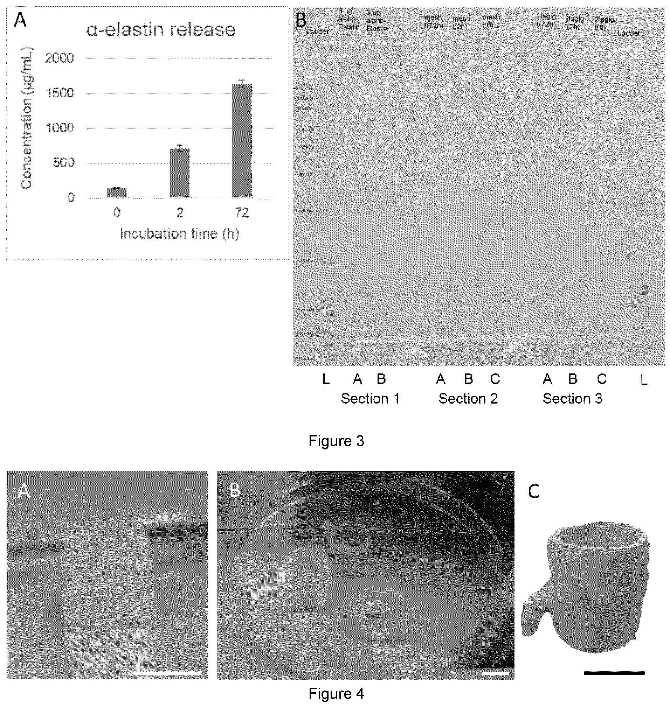

In another approach, soluble elastin was mixed with commercial GelMA (provided by Sigma-Aldrich) and LAP. For this, 0.25 wt% LAP was dissolved in PBS and heated to approx. 60 °C. Subsequently, GelMA was added at 7.5 wt% and stirred until it completely dissolved. After the solution was cooled to approximately 40 °C, soluble elastin (a-elastin or κ-elastin) was added at 7.5 wt% and stirred until it completely dissolved. The bioink preparations were filled in syringes and refrigerated overnight. Prior to printing, the syringes were heated to 25 °C. For $\alpha$-elastin/GelMA and $\kappa$-elastin/GelMA preparations a 18G or 21G blunt needle was used as nozzle, respectively. $10 \times 10$ mm$^2$ one- and two-layer mesh structures were printed as shown in Figure 3 using an INKREDIBLE bioprinter (provided by CELLINK AB, Gothenburg, Sweden). Photocrosslinking was done by UV illumination with the printer's UV modules during the printing process at 365 nm. After printing, UV illumination was continued for 5 min.

Two-layer $\alpha$-elastin/GelMA constructs were incubated in 10 mL PBS and one-layer meshes in 5 mL PBS to study $\alpha$-elastin release. Samples were incubated at 37 °C, 8% $CO_2$ and relative humidity >95%. 200 $\mu$L samples were taken from the supernatant at 0 h, 2 h and 72 h and analyzed by SDS-PAGE and Bradford assay using $\alpha$-elastin as reference.

A substantial amount of elastin peptides was released from the printed constructs after incubation in PBS. The concentration of $\alpha$-elastin peptides released from two-layered constructs was >1.6 $\mu$g/mL (Figure 3A).

In this example it could be shown that by using pristine soluble elastin, biomaterial inks and bioinks with elastin release can be produced.

*Example 8: Bioprinting of vascular replicates*

$\alpha$-ELMA produced according to Example 1 and GelMA were dissolved in PBS at a total protein concentration of 20% (w/v) in a mass ratio of 3:7. LAP was added at a concentration of 0.25% (w/v), the ink was transferred to tainted 3 mL cartridges and subsequently refrigerated overnight for gelation. Hollow cylinders with diameters ranging from 10 mm to 20 mm and a 3D model of a porcine aorta generated from micro-CT data (Figure 4C) were printed on a BIO X device (provided by CELLINK AB, Gothenburg, Sweden) using the temperature-controlled printhead. The printhead was equipped with a 22G nozzle. The temperatures for printhead and printbed were set to 18 °C and 12 °C, respectively, and the extrusion pressure was adjusted in the range of 35 kPa and 40 kPa. Photocuring was performed after each layer with the build-in 405 nm LED module for 1 s in a height of 4 cm above the printed structure. Samples were printed with a height of up to 3 cm (Figures 4A, B).

Example 8 shows that $\alpha$-ELMA/GelMA biomaterial inks are suitable to print stable replicates of anatomical structures. Particular for vascular replicates, the bioprinted structure meets the anatomical proportions using the $\alpha$-ELMA/GelMA biomaterial ink.

*Example 9: Printing of cellularized elastin-based bioinks*

$\alpha$-ELMA and GelMA were prepared as described in Examples 1 and 3, respectively. 0.25% LAP was dissolved in DMEM containing 2% penicillin/streptomycin. The DMEM volume was 90% of the final batch volume.

Subsequently, 5% (w/v) disinfected ($2\times$ 2 h UV irradiation) GelMA was added and stirred at 50 °C until it dissolved. After the batch was cooled to room temperature, 5% disinfected $\alpha$-ELMA (2x 2 h UV irradiation) was added, and the pH was adjusted to neutral by adding NaOH. GFP-labeled HuH7 cells (4$^{th}$ passage, vitality 94%) were suspended in serum and a volume equal to 10% of the total batch volume was mixed with the biomaterial ink through a three-way valve. For negative control, serum without cells was added. For printing, a sterile 22G nozzle was used. The printhead and printbed temperatures were both set to 10 °C, extrusion pressure was adjusted between 20 kPa and 30 kPa, extrusion speed was adjusted between 1.5 mm/s and 2 mm/s and pre- and postflow was set to 10 ms each. For photocuring, the printed structures were illuminated for 120 s using the 405 nm photocuring LED module in the printer. For cultivation, DMEM with penicillin/streptomycin was supplemented by 2 mM stable glutamine and the printed structures were incubated at 37 °C at 8% $CO_2$ and rH >95%.

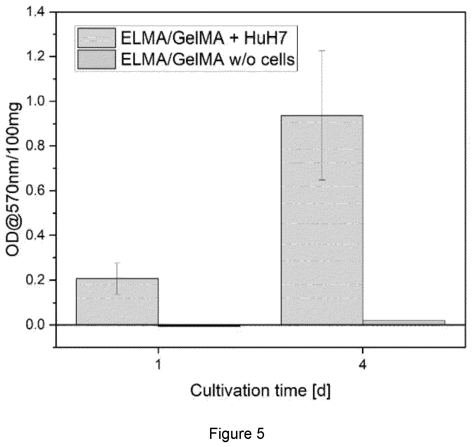

The cell number on days 1 and 4 of cultivation was determined. The cell number increased by a factor of 4 on the fourth day of cultivation compared to the first day in $\alpha$-ELMA/GelMA hydrogels after printing and photocrosslinking (Figure 5).

Example 9 shows that bioinks based on $\alpha$-ELMA/GelMA are cytocompatible both before and after photocuring. Stable structures containing living cells can be printed and cultivated for several days.

**Claims**

1. A process for producing a biocompatible three-dimensional article, the process comprising the steps of

   - providing in an elastin derivative provision step a water-soluble elastin derivative,
   - producing in a hydrogel production step a hydrogel for 3D bioprinting comprising said water-soluble elastin derivative, and
   - 3D bioprinting in a bioprinting step the biocompatible three-dimensional article by extrusion of said hydrogel.

2. The process according to claim 1, wherein the hydrogel further comprises at least one second polymer.

3. The process according to claim 2, wherein the at least one second polymer comprises a polymer of biological origin, a polymer of non-biological origin, or mixtures thereof.

**4.** The process according to claim 3, wherein the polymer of biological origin is selected from the list consisting of polypeptides, polysaccharides, or mixtures thereof, preferably is selected from the list consisting of gelatin, collagen, and mixtures thereof, and most preferably is gelatin.

**5.** The process according to claim 3, wherein the polymer of non-biological origin is a biocompatible polymer of non-biological origin, preferably is a nonionic polyoxyethylene-polyoxypropylene block co-polymer.

**6.** The process according to any of the preceding claims, wherein the water-soluble elastin derivative is hydrolyzed elastin, preferably is α-elastin, β-elastin, κ-elastin, or mixtures thereof.

**7.** The process according to any of the preceding claims, the process further comprising after the elastin derivative provision step the step of

- modifying in an elastin derivative modifying step the water-soluble elastin derivative yielding a modified water-soluble elastin derivative.

**8.** The process according to claim 7, wherein the elastin derivative modifying step comprises the step of

- modifying at least one functional group of the water-soluble elastin derivative yielding a modified functional group.

**9.** The process according to claim 8, wherein the functional group is selected from the list consisting of primary amines ($-NH_2$), secondary amines (-NH-), carbonyl groups (-C=O), hydroxyl groups (-OH), carboxyl groups (-COOH), thiol groups (-SH), phenyl groups ($-C_6H_5$), hydroxyphenyl groups ($-C_6H_5O$) or guanidine groups ($-CH_4N_3$).

**10.** The process according to claim 1, wherein the process further comprises after the step of bioprinting the step of

- crosslinking in a crosslinking step at least a part of the water-soluble elastin derivative.

**11.** The process according to any of the preceding claims 2 to 6, wherein the process further comprises after the step of bioprinting the step of

- crosslinking in a crosslinking step at least a part of the water-soluble elastin derivative, the at least one second polymer, or both.

**12.** The process according to any of the preceding claims 7 to 9, wherein the process further comprises after the step of bioprinting the step of

- crosslinking in a crosslinking step at least a part of the modified water-soluble elastin derivative, the at least one second polymer, or both.

**13.** The process according to the preceding claim 12, wherein the crosslinking step comprises the step of adding a photoinitiator and subsequently photo-activating said photoinitiator, wherein, preferably, the photoinitiator is selected from the list consisting of lithium phenyl(2,4,6-trimethylbenzoyl)phosphinate, 2-(2,4,5,7-tetrabromo-6-oxido-3-oxo-3H-xanthen-9-yl)benzoate, 2-Hydroxy-1-[4-(2-hydroxyethoxy)phenyl]-2-methyl-1-propanone, 2-hydroxy-2-methylpropiophenone, or mixtures thereof, most preferably the photoinitiator is lithium phenyl(2,4,6-trimethylbenzoyl)phosphinate.

**14.** Use of a hydrogel comprising a water-soluble elastin derivative for 3D bioprinting a biocompatible three-dimensional article.

**15.** Article comprising a hydrogel comprising a water-soluble elastin derivative.

**Figures**

Figure 1

Figure 2

Figure 3

Figure 4

Figure 5

Europäisches
Patentamt

European
Patent Office

Office européen
des brevets

# EUROPEAN SEARCH REPORT

Application Number

**EP 22 19 5467**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/191897 A1 (COLLPLANT LTD [IL]) 30 September 2021 (2021-09-30) * page 48, line 30 - page 49, line 4 * * page 50, line 20 - page 52, line 32 * * page 55, lines 10-13 * * page 59, lines 5-10 * * page 62, lines 12-19 * * examples 1-4 * * claims 1-50 * | 1-15 | INV. A61L27/22 A61L27/26 A61L27/52 A61L27/56 B33Y10/00 |
| X | SOHYUNG LEE ET AL: "Human-Recombinant-Elastin-Based Bioinks for 3D Bioprinting of Vascularized Soft Tissues", ADVANCED MATERIALS, VCH PUBLISHERS, DE, vol. 32, no. 45, 1 October 2020 (2020-10-01), page n/a, XP071877536, ISSN: 0935-9648, DOI: 10.1002/ADMA.202003915 * the whole document * | 1-4,6-15 | |
| X | CN 112 206 358 A (SUZHOU NUOPU REGENERATIVE MEDICINE CO LTD) 12 January 2021 (2021-01-12) * claims 1-10; examples 1-6 * | 1-4,6, 10-12, 14,15 | TECHNICAL FIELDS SEARCHED (IPC) A61L B33Y |

The present search report has been drawn up for all claims

| Place of search | Date of completion of the search | Examiner |
|---|---|---|
| The Hague | 21 February 2023 | Burtan, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                    

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 5467

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2023

| Patent document cited in search report | | Publication date | Patent family member(s) | | Publication date |
|---|---|---|---|---|---|
| WO 2021191897 | A1 | 30-09-2021 | AU | 2021243961 A1 | 24-11-2022 |
| | | | BR | 112022018883 A2 | 13-12-2022 |
| | | | CA | 3175869 A1 | 30-09-2021 |
| | | | CN | 115605235 A | 13-01-2023 |
| | | | EP | 4126088 A1 | 08-02-2023 |
| | | | IL | 296636 A | 01-11-2022 |
| | | | KR | 20230004525 A | 06-01-2023 |
| | | | WO | 2021191897 A1 | 30-09-2021 |
| CN 112206358 | A | 12-01-2021 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3581212 A1

**Non-patent literature cited in the description**

- *Advanced Materials,* 2020, vol. 32 (45), 1-10
- *ACS omega,* 2021, vol. 6 (33), 21368-21383
- **YUE K ; LI X ; SCHROBBACK K ; SHEIKHI A ; AN-NABI N ; LEIJTEN J ; ZHANG W ; ZHANG YS ; HUTMACHER DW ; KLEIN TJ.** Structural analysis of photocrosslinkable methacryloyl-modified protein derivatives. *Biomaterials,* 29 May 2017, vol. 139, 163-171